# EUROPEAN PATENT APPLICATION

(11) **EP 2 835 138 A1**
(43) Date of publication of application: **11.02.2015**
(21) Application number: 14179743.1
(22) Date of filing: 04.08.2014
(51) Int. Cl.: A61L 15/58

(54) **Adhesive and use for attachment to microfiber garments**

(30) Priority: 05.08.2013 US 201361862264 P
(71) Applicant: McNeil-PPC, Inc., Skillman, New Jersey 08558 (US)
(72) Inventor: Austin,, Jennifer Jensen, Texas, TX 77382 (US); Quinn,, Thomas Harry, Minnesota, MN 55105 (US)
(74) Representative: Carpmaels & Ransford LLP

(57) **Abstract**

The present invention relates to an adhesive and its use for attachment to microfiber garments. The adhesive provides peel strength, complex modulus and glass transition properties suitable for this application.

## Description

### Field of the Invention

The present invention relates to an adhesive and its use for attachment of a substrate or article to microfiber garments. The adhesive provides peel strength, loss modulus and storage modulus properties suitable for this application.

### Background of the Invention

It is well known to utilize absorbent articles such as sanitary pads and liners in underwear to absorb bodily fluids. Most commonly, the absorbent articles have one surface with a positioning adhesive applied to it to attach the article to the underwear. Underwear has changed over time from pure cotton fiber to cotton/polymer fiber blends. The size of the fibers has also changed to provide improved comfort. Recently, microfibers have been introduced in underwear.

As a result of the change in underwear materials, conventional positioning adhesives are not effective in holding absorbent articles in place. Therefore, there is a need for an adhesive that functions well as a positioning adhesive for absorbent articles.

United States Patent No. 7,842,022, herein incorporated by reference in its entirety, teaches adhesives that are applied to a reference microfiber substrate. The adhesive survives a first peel force of at least 0.6 N per 5 cm when measured from a microfiber substrate. The adhesives exemplified for bonding to microfibers are hot melt silicone pressure sensitive adhesives. A second adhesive is also taught. The second adhesive is conventional in that it bonds well to conventional cotton fibers, but not to microfibers.

Therefore, despite the disclosure of the reference, there is a continuing need for an adhesive with suitable properties for attaching absorbent articles to garments.

### Summary of the Invention

In one embodiment, the present invention relates to an adhesive used alone (or in combination with other adhesives) adapted to or suitable for adhering an article to microfiber to both cotton fiber and microfiber wherein the adhesive has, as measured by DMA testing method described below in Example 3: a) a complex modulus (G*), at 25°C, a shear stress of 1000 Pa. and a frequency of 6.3 rad/sec., ranging from about 1000 to about 20000 Pa; b) a glass transition (Tg) temperature, at a frequency of 6.3 rad/sec. and shear stress of 1000 Pa., of less than about 10°C; or c) a combination thereof.

In another embodiment, the present invention relates to an adhesive adapted to or suitable for adhering an article to microfiber wherein the adhesive has, as measured by DMA testing method described below in Example 3: a) a loss modulus (G"), at 25°C, a shear stress of 1000 Pa. and a frequency of 6.3 rad/sec., ranging from about 1000 to about 15000 Pa; b) a storage modulus (G') at 25°C, shear stress of 1000 Pa. and frequency of 6.3 rad/sec., ranging from about 1000 to about 15000 Pa; or c) a combination thereof.

In certain embodiments, the present invention relates to a disposable absorbent article for personal hygiene, the article having a wearer-facing surface and a garment facing surface, the garment facing surface comprising an adhesive having, as measured by DMA testing method described below in Example 3: a) a complex modulus (G*), at 25°C, a shear stress of 1000 Pa. and frequency of 6.3 rad/sec., ranging from about 1000 to about 20000 Pa; b) a glass transition (Tg) temperature, at a frequency of 6.3rad/sec. and shear stress of 1000 Pa., of of less than about 10°C; or c) a combination thereof.

In certain embodiments, the present invention relates to methods of adhering an article or substrate to microfiber comprising the steps of:
a) providing a microfiber; and
b) attaching to the microfiber an article or substrate containing an adhesive such that the article or substrate is attached to the microfiber by the adhesive, wherein the adhesive has, as measured by DMA testing method described below in Example 3: i) a complex modulus (G*), at 25°C, a shear stress of 1000 Pa. and a frequency of 6.3 rad/sec., ranging from about 1000 to about 20000 Pa; ii) a glass transition (Tg) temperature, at a frequency of 6.3 rad/sec. and shear stress of 1000 Pa., of less than about 10°C; or iii) a combination thereof.

In certain embodiments, the present invention relates to an article formed, in use, comprising:
a) a microfiber; and
b) an adhesive in contact with the microfiber, having, as measured by DMA testing method described below in Example 3: i) a complex modulus (G*), at 25°C, a shear stress of 1000 Pa. and a frequency of 6.3 rad/sec., ranging from about 1000 to about 20000 Pa; ii) a glass transition (Tg) termperature, at a frequency of 6.3 rad/sec. and shear stress of 1000 Pa., of less than about 10°C; or iii) a combination thereof.

In another embodiment, the present invention further relates to a disposable absorbent article for personal hygiene, the article having a wearer-facing surface and a garment facing surface, the garment facing surface comprising an adhesive having a peel strength to microfibers ranging from about 1.96 to about 4.9 N at an adhesive coat weight of 20 g/m², as measured by T-Peel testing method described below in Example 2 from a microfiber substrate comprising 95% polyamide and 5% elastane (LYCRA), optionally, having a thickness of 0.48 mm and, optionally, having a basis weight of about 170 g/m², (optionally, between 161.5 and 178.5 g/m²) wherein the adhesive is substantially free of silicone.

The present invention still further relates to a disposable absorbent article for personal hygiene, the article having a wearer-facing surface and a garment facing surface, the garment facing surface comprising an adhesive, the adhesive comprising a first polymer having a melt index of from about 12 grams per 10 minutes to about 30 grams per 10 minutes and a second polymer having a melt index of less than about 12 grams per 10 minutes, where the melt index, in each case, is measured using ASTM Method D 1238, Condition G.

The complex modulus, at 25°C, may range from about 1000 to about 15000 Pa, or from about 2000 to about 10000 Pa. The glass transition temperature may be less than about 5°C, or less than about 2°C.

The adhesive may comprise a first polymer. The first polymer may be a styrenic block copolymer. The styrenic block copolymer may comprise a styrenic tri-block polymer and may further comprise a styrenic di-block polymer, forming a styrenic tri-block/diblock polymer mixture.

The present invention still further relates to a disposable absorbent article for personal hygiene, the article having a wearer-facing surface and a garment facing surface, the garment facing surface comprising an adhesive, wherein the adhesive comprises a first polymer wherein the first polymer is a styrenic block copolymer comprising:
i. a styrenic tri-block polymer; and
ii. a styrenic di-block polymer
to form a styrenic tri-block/diblock polymer mixture.

The styrenic di-block polymer may be present at a concentration of at least 50%, by weight of the styrenic tri-block/diblock polymer mixture, or may be present at a concentration of at least 60%, by weight of the styrenic tri-block/diblock polymer mixture, or may be present at a concentration of at least 70%, by weight of the styrenic tri-block/diblock polymer mixture.

The first polymer may have a melt index of greater than about 12 grams per 10 minutes as measured by ASTM Method D 1238, Condition G.

The adhesive may further comprise a second polymer. The second polymer may have a melt index less than about about 12 grams per 10 minutes as measured by ASTM Method D 1238, Condition G. The second polymer may be a styrene-butadiene polymer.

The article may be an incontinence product, panty liner or feminine napkin.

### Detailed Description of the Invention

The adhesives and articles of the present invention can comprise, consist of, or consist essentially of the essential elements and limitations of the invention described herein, as well any of the additional or optional ingredients, components, or limitations described herein. The term "comprising" (and its grammatical variations) as used herein is used in the inclusive sense of "having" or "including" and not in the exclusive sense of "consisting only of."

The terms "a" and "the" as used herein are understood to encompass the plural as well as the singular.

Unless otherwise indicated, all documents cited are, in relevant part, incorporated herein by reference; the citation of any document is not to be construed as an admission that it is prior art with response to the present invention. Furthermore, all documents incorporated herein by reference in their entirety are only incorporated herein to the extent that they are not inconsistent with this specification.

As used herein, the term "microfiber" means fibers, or fabrics containing fibers, having a denier of not more than 1 (1 denier=1 g/9000 m of fiber) and a diameter in cross section of not more than 10 µm. Microfibers are artificial man-made fibers and most typically consist of polyester or polyamides, such as nylon. Microfibers are used by the fabric industry for making very fine close woven fabric materials and knitted fabrics, which are characterized by soft handle and breathability. Microfiber fabrics have very different physical characteristics compared to conventional cotton fabrics. Oftentimes microfiber garments also contain elastan/LYCRA fibers for providing elasticity. Due to the small diameter of the microfibers the density of the fabrics made therefrom is very high compared to the one of cotton fabrics. Thanks to the small fiber diameter of those microfibers the void space between the individual microfibers is very low compared to cotton fabrics. Commercially available microfiber materials are marketed by, for example, DuPont under the trade name TACTEL® or by Nylstar under the trade name MERYL®.

As used herein, the term "non transferable to cotton" means that the adhesive does not leave residue (or residue is not present) on cotton (as determined by visual inspection after removing the absorbent article from the garment)

As used herein, the term "absorbent article", in a very broad sense, means any article being able to receive and/or absorb and/or contain and/or retain fluids and/or exudates, especially bodily fluids/bodily exudates. The absorbent article, which is referred to in the present invention typically comprises a fluid pervious cover layer as the wearer-facing surface, a fluid impervious barrier layer as the garment-facing surface that is preferably water vapour and/or gas pervious and an absorbent core comprised there between. Furthermore, absorbent articles in the context of the present invention are provided with a means for their attachment to the user's garment, in particular with an adhesive. In certain embodiments, absorbent articles in the context of the present invention are disposable absorbent articles. Typical disposable absorbent articles according to the present invention are absorbent articles for personal hygiene, such as baby care articles like baby diapers; incontinence pads and perspiration pads like underarm sweat pads or hat bands and such disposable absorbent articles as those used for feminine hygiene like catamimials in the form of incontinence products, sanitary napkins and/or panty liners. In certain embodiments, disposable absorbent article is a sanitary napkin or panty liner.

As used herein, the term "disposable" means articles which are not intended to be laundered or otherwise restored or reused as an article (i.e. they are intended to be discarded after a single use and preferably to be recycled, composted or otherwise disposed of in an environmentally compatible manner).

As used herein, the phrase "visual inspection" means that a human viewer can visually discern the presence of residue with the unaided eye (excepting standard corrective lenses adapted to compensate for near-sightedness, farsightedness, or stigmatism, or other corrected vision) in lighting at least equal to the illumination of a standard 100 watt incandescent white light bulb at a distance of 0.25 meter.

As used herein, the terms "body fluid" or "bodily fluid" means any fluid produced by the human or mammalian body including for instance perspiration, urine, blood, menstrual fluids, vaginal secretions and the like.

As used herein, the term "in use", refers to the period of time that starts when the absorbent article is actually put in contact with the anatomy of the user (i.e., wearer).

As used herein, the term "glass transition" or "Tg" means the temperature indicating the relaxation in a polymer where a material changes from a glass to a rubber and is determined by tensile dynamic mechanical analysis (DMA) performed in the linear elastic region of the material using parallel plate geometry with a gap of 1 mm at a frequency of 6.3 rad/sec., using a temperature ramp method at a temperature ramp rate of about 1-2° C./min or slower. The Tan delta peak temperature is taken as the glass transition (Tg) of the particular material or phase.

As said infra, an absorbent article in the context of the present invention comprises the adhesive of the present invention for the attachment of this article to the user's garments. The adhesive of the present invention in this context is also referred to as "panty fastening adhesive" or "PFA". The PFA is provided on the garment facing surface of the absorbent article of the present invention, typically the backsheet, for attaching said article to the garment of a wearer. Similarly, if the product is a winged product, the wings can also be provided with PFA on the garment-facing surface in order to secure the wings to the wearer's garment.

"Surface coverage" of a surface as used herein means that said surface is covered by a material to a certain percentage. For instance, 30% PFA surface coverage of the garment facing surface of the absorbent article herein means that 30% of the total garment-facing surface of the absorbent article is covered with PFA. The garment-facing surface of the article of the present invention can be covered with the PFA in a continuous manner, i.e. a continuous coating of the whole garment-facing surface, or in a discontinuous manner, such as by stripes, dots or very fine droplets of PFA. For surface coverage herein the part of the garment-facing surface of the article, which is actually covered by PFA material is taken.

In certain embodiments the adhesive of the present invention has a surface coverage on the garment-facing surface of the article of at least 30%, optionally 40%, optionally 50%, optionally 60% or optionally 70-100% in order to achieve sufficient PFA-coated area on the garment-facing surface of the absorbent article of the present invention for making the bond to the garment material.

All percentages, parts and ratios are based upon the total weight of the adhesive of the present invention, unless otherwise specified.

### Adhesive

The present invention comprises an adhesive adapted to or suitable for adherence to a microfibers.

In certain embodiments, the adhesives are thermoplastic. As used herein the term "thermoplastic" means an adhesive that repeatedly is transferable to a liquid or semi-liquid state by heating, and to a form-stable, essentially elastic state by cooling, within a temperature range specific for the adhesive.

In certain embodiments, the adhesives of the present invention are free or substantially free of crosslinking. The term "substantially free of crosslinking", as used herein, means that a substantial amount of the polymers in the adhesive have not undergone crosslinking, as a result of crosslinking treatment or otherwise, and, therefore, do not suffer from restricted molecular mobility which may tend to limit the extension of the polymer under loading. Accordingly, the adhesives of the present invention are also essentially free of crosslinking agents. "Essentially free" as used with respect to cross-link agents is defined as adhesives having less than 5% (or about 5%), optionally, 3% (or about 3%), optionally, 1% (or about 1 %), optionally, 0.5% (or about 0.5%), optionally, 0.1 % (or about 0.1 %), optionally, 0.01 % (or about 0.01 %) or zero percent, by weight of the total adhesive, of crosslinking agents. Commonly used crosslinking agents are detailed in US Patent No. 8,481,645, herein incorporated by reference in its entirety.

### First polymer

The adhesive of the present invention comprises a first polymer either alone or in combination with one or more additional polymers. In certain embodiments, the first polymer is a soft or elastomer polymer/copolymer. The terms polymer and copolymer are used interchangeably as they relate to the soft or elastomer polymer. In one embodiment, the first polymer is incorporated (optionally, in combination with one or more additional polymers or additives [as described below]) such that the adhesive according to the present invention, when measured by DMA testing method described below in Example 3, has a complex modulus (G*) at 25°C and shear stress of 1000 Pa. when measured at a frequency of 6.3 rad/sec., ranging from 1000 (or about 1000) to 20000 (or about 20000), optionally, from 1000 (or about 1000) to 15000 (or about 15000), optionally, from 2000 (or about 2000) to 10000 (or about 10000) Pa., or optionally, from 2000 (or about 2000) to 8000 (or about 8000) Pa.. In another embodiment, the first polymer is incorporated (optionally, in combination with one or more additional polymers or additives [as described below]) such that the adhesive according to the present invention, when measured by DMA testing method described below in Example 3, has a glass transition (Tg) temperature when measured at a frequency of 6.3 rad/sec. and shear stress of 1000 Pa., of less than 10°C (or about 10°C), optionally, less than 5°C (or about 5°C), or optionally, less than 2°C (or about 2°C) so long as, in each case, the lower limit is no lower than -10°C (or about -10°C). In still another embodiment, the first polymer is incorporated (optionally, in combination with one or more additional polymers or additives [as described below]) such that the adhesive according to the present invention has both a complex modulus (G*) value and a glass transition (Tg) value falling within the respective ranges indicated above.

In certain embodiments, the first polymer is incorporated (optionally, in combination with one or more additional polymers or additives [as described below]) such that the complex modulus (G*) of the adhesive according to the present invention, when measured, by DMA testing method described below in Example 3, across a temperature range starting from 25°C to 40°C, at a frequency of 6.3 rad/sec. and shear stress of 1000 Pa., has an average decline (i.e., the average difference in the resultant complex modulus (G*) value of the adhesive [at 40°C] from the initial complex modulus (G*)value of the adhesive [at 25°C]), of more than 25%, optionally more than 30%, optionally more than 40%, optionally more than 50%, more than 55%.

In certain embodiments, the first polymer is incorporated (optionally, in combination with one or more additional polymers or additives [as described below]) such that the adhesive according to the present invention, when measured by DMA testing method described below in Example 3, has a loss modulus (G"), at 25°C, a shear stress of 1000 Pa. and at a frequency of 6.3 rad/sec., ranging from 1000 (or about 1000) to 15000 (or about 15000), optionally, from 1000 (or about 1000) to 10000 (or about 10000), or optionally, from 2000 (or about 2000) to 7000 (or about 7000) Pa.

In certain embodiments, the first polymer is incorporated (optionally, in combination with one or more additional polymers or additives [as described below])such that the loss modulus (G") of the adhesive according to the present invention, when measured, by DMA testing method described below in Example 3, across a temperature range starting from 25°C to 40°C, at a frequency of 6.3 rad/sec. and shear stress of 1000 Pa., has an average decline (i.e., the average difference in the resultant loss modulus (G") value of the adhesive [at 40°C] from the initial loss modulus (G") value of the adhesive [at 25°C]), of more than 25%, optionally more than 30%, or optionally more than 35%.

In certain embodiments, the first polymer is incorporated (optionally, in combination with one or more additional polymers or additives [as described below]) such that the adhesive according to the present invention, when measured by DMA testing method described below in Example 3, has a storage modulus (G'), at 25°C, a shear stress of 1000 Pa. and at a frequency of 6.3 rad/sec., ranging from 1000 (or about 1000) to 15000 (or about 15000), optionally, from 1000 (or about 1000) to 10000 (or about 10000), or optionally, from 2000 (or about 2000) to 7000 (or about 7000) Pa.

In certain embodiments, the first polymer is incorporated (optionally, in combination with one or more additional polymers or additives [as described below])such that the storage modulus (G') of the adhesive according to the present invention, when measured, by DMA testing method described below in Example 3, across a temperature range starting from 25°C to 40°C, at a frequency of 6.3 rad/sec. and shear stress of 1000 Pa., has an average decline (i.e., the average difference in the resultant storage modulus (G') value of the adhesive [at 40°C] from the initial storage modulus (G') value of the adhesive [at 25°C]), of more than 25%, optionally more than 35%, or optionally more than 45%.

In certain embodiments, the first polymer is incorporated (optionally, in combination with one or more additional polymers or additives [as described below]) such that the adhesive according to the present invention has a peel strength to microfibers ranging from 1.96 (or about 1.96) to 4.9 (or about 4.9), optionally, from 1.96 (or about 1.96) to 3.92 (or about 3.92), or optionally, from 2.45 (or about 2.45) to 3.43 (or about 3.43) N at an adhesive coat weight of 20 g/m².

In certain embodiments, the first polymer is or comprises a styrenic block copolymer. Examples of styrenic block copolymers include, but are not limited to, (or comprise) styrenic triblock polymers such as styrene-isoprene-styrene tri-block copolymers. As is known in the art, tri-block polymers (isoprene-styrene-isoprene) are a mixture of polymers comprising some di-block polymer (styrene-isoprene) due to the nature of the polymerization reaction. In certain embodiments, adhesives of the present invention utilize a tri-block/di-block polymer mixture having di-block polymer concentration of at least about 50%, optionally, at least 55% (or about 55%), optionally, from 60% (or about 60%) to 70% (or about 70%) or, optionally, 66% (or about 66%), by weight of styrenic tri-block/diblock polymer mixture. Suitable styrene-isoprene-styrene tri-block mixtures include the KRATON brand such as KRATON D-1113 (16% styrene, linear/55% diblock), KRATON D-1119 (22% styrene, linear/65% diblock) (each supplied by Kraton Polymers, Houston, TX); VECTOR 4186A radial block copolymer (18% styrene /75% diblock) supplied by Dexco Polymers, Houston, TX; or mixtures thereof. The total amount of this tri-block/diblock polymer mixture in the adhesives of the present invention is from 10% (or about 10%) to 50% (or about 50%), optionally, from 20% (or about 20%) to 40% (or about 40%), or optionally from 25% (or about 25%) to 35% (or about 35%), by weight, of the total adhesive.

In certain embodiments, the first polymer has a melt index of greater than 12 (or about 12) grams per 10 minutes, optionally from 12 (or about 12) grams per 10 minutes to 30 (or about 30) grams per 10 minutes, optionally from 15 (or about 15) grams per 10 minutes to 25 (or about 25) grams per 10 minutes, or optionally from 20 (or about 20) grams per 10 minutes to 25 (or about 25) grams per 10 minutes, as measured by ASTM Method D 1238, Condition G. Examples include KRATON D 1113 (melt index of about 24 g/10 min.) and KRATON D 1119 (melt index of about 25 g/10 min.).

### Tackifying Resin

The adhesive of the present invention also comprises at least one tackifying resin. Useful tackifying resins or mixture of such resins include those having a softening point of from 85°C (or about 85°C) to 120°C (or about 120°C), optionally, from 90°C (or about 90°C) to 110°C (or about 110°C), or optionally, from 90°C (or about 90°C) to 105°C (or about 105°C) may be used. Suitable commercially available tackifying resins include hydrocarbon resins such as ESCOREZ 5400 (cycloaliphatic hydrocarbon resin; softening point = 100°C) from ExxonMobil Chemical Company, EASTOTAC H100W (softening point = 100°C) and REGALREZ 1094 (softening point = 94°C) each from Eastman Chemical, Kingsport, TN; hydrogenated rosin such as FORAL AX from Pinova, Inc., Brunswick, GA; rosin derivatives such as SYLVALITE RE100L (softening point = 100°C) from Arizona Chemical, Jacksonville, FL; polyterpenes such as SYLVARES TR 7115 (softening point = 115°C) from Arizona Chemical; and C5 hydrocarbons such as WINGTACK 95 (softening point = 95°C) from Cray Valley, Exton, PA and other tackifiers.

In certain embodiments, liquid tackifiers having a softening point of from 5°C (or about 5°C) to 40°C (or about 40°C), optionally, from 5°C (or about 5°C) to 30°C (or about 30°C), or optionally, from 10°C (or about 10°C) to 20°C (or about 20°C) are used in the adhesives of the present invention. Examples of suitable liquid tackifiers include, but are not limited to, REGALREZ 1018 (softening point = 18°C) from Eastman Chemical, Kingsport, TN, WINGTACK 10 (softening point = 10°C).

In some embodiments, liquid rubbers such INDOPOL and polybutene (INEOS, Switzerland) may also be used as the tackifying resin.

In certain embodiments, the tackifying resin is a hydrocarbon resin. In other embodiments, the tackifying resin is a cycloaliphatic hydrocarbon resin. The amount of tackifying resin may range from 40% (or about 40%) to 70% (or about 70%), optionally from 45% (or about 45%) to 65% (or about 65%), or optionally from 50% (or about 50%) to 60% (or about 60%), by weight, of the total adhesive.

### Plasticizer

The adhesive of the present invention also comprises at least one plasticizer. Suitable plasticizers include hydrogenated cycloaliphatic petroleum hydrocarbons, such as REGALREZ® 1018 from EASTMAN Kingsport, TN; and mineral oil or paraffinic oil, such as KRYSTOL® 550 from Petro-Canada Lubricants Company, Mississauga, ON, Canada or naphthenic oils such as NYFLEX 222B from Nynas AB, Stockholm, Sweden. In certain embodiments, the plasticizer is a naphthenic oil. When utilized, the amount of the plasticizer may range from about 10% to about 30%, optionally from about 15% to about 25%, or optionally from about 15% to about 20%, by weight, of the total adhesive.

### Second Polymer

In certain embodiments, the adhesive of the present invention may further contain a second polymer in addition to the above mentioned (or, in this case, first) polymer. Polymers suitable for use as the second polymer include, but is not limited to styrene-butadiene polymers such as SOLPRENE 1205 (18% wt. styrene) from Dynasol Company, Houston, TX and KRATON D1118 from Kraton Polymers, Houston, TX; and mixtures thereof. In certain embodiments, the second polymer is a styrene-butadiene polymer. A more detailed description of styrene butadiene block polymers can be found in US Patent 8,378,015, herein incorporated by reference in its entirety.

In certain embodiments, the second polymer generally has a has a melt index of less than or equal to 12 (or about 12) grams per 10 minutes, optionally, from 1 (or about 1) grams per 10 minutes to 12 (or about 12) grams per 10 minutes, or optionally from 2 (or about 2) grams per 10 minutes to 10 (or about 10) grams per 10 minutes, as measured by ASTM Method D 1238, Condition G. An example of a polymer falling within the melt index described in this paragraph is KRATON D1118 (melt index of about 10 g/10 min.).

When utilized, the amount of the second polymer may range from about 5% to about 20%, optionally from about 5% to about 15%, or optionally from about 5% to about 10%, by weight, by weight of the total adhesive.

Optionally, the adhesive of the present invention may also incorporate conventional adhesive compounding ingredients, such as stabilizers. Suitable stabilizers include, but are not limited to, phenolic antioxidants such as hindered phenolic antioxidants, BNX-1010 or BNX-1076 from MAYZO Company Norcross, GA or mixtures of such phenolic antioxidants. In certain embodiments, the stabilizer is a hindered phenolic antioxidant. The stabilizer may be added at amounts known in the art, for example from about 0.25% to about 2% by weight or, optionally, 1% (or about 1%) by weight. The adhesive can be applied by die coating or other means known in the art. Slot die coating is a continuous coating process that involves passing the adhesive through a die onto the backing.

In certain embodiments, the adhesives according to the present invention have a peel strength to microfibers ranging from 1.96 (or about 1.96) to 4.9 (or about 4.9), optionally, from 1.96 (or about 1.96) to 3.92 (or about 3.92), or optionally, from 2.45 (or about 2.45) to 3.43 (or about 3.43) N at an adhesive coat weight of 20 g/m², using the T-Peel testing method described below in Example 2 from a microfiber substrate

In certain embodiments, the adhesives according to the present invention, when measured by DMA testing method described below in Example 3, have a loss modulus, at 25°C, a shear stress of 1000 Pa. and at a frequency of 6.3 rad/sec., ranging from 1000 (or about 1000) to 15000 (or about 15000), optionally, from 1000 (or about 1000) to 10000 (or about 10000), or optionally, from 2000 (or about 2000) to 7000 (or about 7000) Pa.

In certain embodiments, the adhesives according to the present invention, when measured by DMA testing method described below in Example 3, have a storage modulus, at 25°C and at a frequency of 10 rad/sec., ranging from 1000 (or about 1000) to 15000 (or about 15000), optionally, from 1000 (or about 1000) to 10000 (or about 10000), or optionally, from 2000 (or about 2000) to 7000 (or about 7000) Pa.

In certain embodiments, the adhesives according to the present invention are non transferable to cotton as observed by visual inspection.

In certain embodiments, when applied to the backing or barrier layer, the adhesive of the present invention is applied at a coating weight of from about 1 g/m² to about 35 g/m², optionally from about 10 g/m² to about 30 g/m², or optionally from about 15 g/m² to about 25 g/m².

In still certain embodiments, the adhesives of the present invention are free of or essentially free of silicone. "Essentially free" as used with respect to silicone is defined as adhesives having less than 5% (or about 5%), optionally, 3% (or about 3%), optionally, 1% (or about 1 %), optionally, 0.5% (or about 0.5%), optionally, 0.1% (or about 0.1%), optionally, 0.01 % (or about 0.01 %) or zero percent, by weight of the total adhesive, of silicone.

Without being limited by theory, it is believed that the presence of silicone negatively affects the adhesion properties (or stickiness) of the adhesive. Generally, the silicone compounds migrate to the adhesive surface and produce a slippery feel as the adhesive surface.

The adhesives of the present invention exhibit adherence properties upon formation of the formulation (as described in Example 1 below) and do not require activation, whether by temperature or otherwise.

### Absorbent Articles

The adhesives of the present invention may be applied to absorbent articles that are applied to microfibers or garments comprising microfibers. The absorbent article typically contains a cover layer having a wearer-facing surface, optionally an absorbent core and a barrier layer having a garment-facing surface.

### Cover Layer

The cover layer may be made of a relatively low density, bulky, high-loft non-woven web material. The cover layer may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. The cover layer may have a basis weight in the range of about 10 g/m² to about 75 g/m².

Bi-component fibers may be made up of a polyester layer and a polyethylene sheath. The use of appropriate bi-component materials results in a fusible non-woven fabric. Examples of such fusible fabrics are described in U.S. Patent No. 4,555,430 issued Nov. 26, 1985 to Chicopee, herein incorporated by reference in its entirety. Using a fusible fabric increases the ease with which the cover layer may be mounted to the absorbent layer and/or to the barrier layer.

The cover layer has a wearer-facing surface which, when the absorbent article is in use, contacts the user (i.e., wearer). In certain embodiments, the cover layer has a relatively high degree of wettability, although the individual fibers comprising the cover may not be particularly hydrophilic. The cover material should also contain a great number of relatively large pores. This is because the cover layer is intended to take-up body fluid rapidly and transports it away from the body and the point of deposition. Therefore, the cover layer contributes little to the time taken for the absorbent article to absorb a given quantity of liquid (penetration time).

The cover layer may be treated to allow fluid to pass through it readily. The cover layer also functions to transfer the fluid quickly to the underlying layers of the absorbent article. Thus, the cover layer is advantageously wettable, hydrophilic and porous. When composed of synthetic hydrophobic fibers such as polyester or bi-component fibers, the cover layer may be treated with a surfactant to impart the desired degree of wettability.

The cover may be made from a 16 g/m² thermal bonded polypropylene fiber nonwoven of the type commercially available from Polystar Company, Salvador, BA , Brazil under product code 142250. Alternatively, the cover layer can also be made of polymer film having large pores. Because of such high porosity, the film accomplishes the function of quickly transferring body fluid to the underlying layers of the absorbent article. A suitable cover material of this type is commercially found on the STAYFREE Dry Max Ultrathin product distributed by McNeil-PPC, Inc.

The cover layer may be embossed to the remainder of the absorbent core in order to aid in promoting hydrophilicity by fusing the cover to the next layer. Such fusion may be effected locally, at a plurality of sites or over the entire contact surface of cover layer and absorbent core. Alternatively, the cover layer may be attached to the absorbent core by other means such as by adhesion.

### Transfer Layer

In certain embodiments, adjacent to the cover layer on its inner side and bonded to the cover layer may be a transfer layer. The transfer layer provides the means of receiving body fluid from the cover layer and holding it until the underlying absorbent core has an opportunity to absorb the fluid, and therefore acts as a fluid transfer or acquisition layer. The transfer layer is, preferably, more dense than and has a larger proportion of smaller pores than the cover layer. These attributes allow the transfer layer to contain body fluid and hold it away from the outer side of the cover layer, thereby preventing the fluid from rewetting the cover layer and its surface. However, the transfer layer is, preferably, not so dense as to prevent the passage of the fluid through the layer into the underlying absorbent core.

The transfer layer may be composed of fibrous materials, such as wood pulp, polyester, rayon, flexible foam, or the like, or combinations thereof. The transfer layer 43 may also comprise thermoplastic fibers for the purpose of stabilizing the layer and maintaining its structural integrity. The transfer layer 43 may be treated with surfactant on one or both sides in order to increase its wettability, although generally the transfer layer is relatively hydrophilic and may not require treatment. The transfer layer is preferably bonded or adhered on both sides to the adjacent layers, i.e. the cover layer and the underlying absorbent core. Examples of suitable materials for the transfer layer are through air bonded pulp sold by Buckeye of Memphis, Tenn., under the designation VIZORB 3008, which has a basis weight of 110 g/m², VIZORB 3042, which has a basis weight of 100 g/m², VIZORB 3010, which has a basis weight of 90 g/m² and others.

### Absorbent Core

The absorbent article may have and absorbent core which is a blend or mixture of cellulosic fibers and superabsorbent disposed therein. Cellulosic fibers that can be used in the absorbent core are well known in the art and include wood pulp, cotton, flax and peat moss. Wood pulp is preferred. Pulps can be obtained from mechanical or chemi-mechanical, sulfite, Kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Patent No. 5,916,670 to improved flexibility of the product, which patent is herein incorporated by reference in its entirety. The flexibility of the material may also be improved by mechanically working the material or tenderizing the material.

The absorbent core can contain any superabsorbent polymer (SAP), which are well known in the art. For the purposes of the present invention, the term "superabsorbent polymer" (or "SAP") refers to materials, which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc.

The absorbent core may include between 50% and 100% cellulose pulp by weight and 0% and 50% superabsorbent polymer by weight. In one example, the absorbent core is constructed from about 93 % fluff pulp by weight, suitable pulp commercially available as Golden Isles Fluff Pulp 420#HD 7% Moisture, from GP Cellulose, Brunswick, Georgia, USA, mixed with about 7% superabsorbent polymer by weight, suitable SAP commercially available as Aqua Keep SA70N from Sumitomo Seika Chemicals Co., Ltd., Osaka, Japan.

### Barrier Layer

A barrier layer has a garment facing surface and is used to prevent liquid that is entrapped in the absorbent article from egressing the sanitary napkin or liner and staining the wearer's garment (e.g., undergarment). The barrier layer is preferably made of polymeric film, although it may be made of liquid or fluid impervious, air-permeable material fluid repellent-treated non-woven or micropore films or foams.

The barrier layer may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable barrier. The cover layer and the barrier layer may be joined along their marginal portions so as to form an enclosure or flange seal that maintains the absorbent core captive. The joint may be made by means of adhesives, heat-bonding, ultrasonic bonding, radio frequency sealing, mechanical crimping, and the like and combinations thereof.

### Other Optional Components

Absorbent articles utilized in the present invention may or may not include wings, flaps or tabs for securing the absorbent article to an undergarment. Wings, also called, among other things, flaps or tabs, and their use in sanitary protection articles is described in U.S. Patent No. 4,687,478 to Van Tilburg; U.S. Patent No. 4,589,876 also to Van Tilburg; U.S. Patent No. 4,900,320 to McCoy; and U.S. Patent No. 4,608,047 to Mattingly, each of which patents is herein incorporated by reference in its entirety. As disclosed in the above documents, wings are generally speaking flexible and configured to be folded over the edges of the underwear so that the wings are disposed between the edges of the underwear.

The absorbent article utilized with the present invention may be applied to the crotch of the microfiber containing garment by placing the garment-facing surface against the inside surface of the crotch of the garment.

Any or all of the cover, absorbent layer, transfer layer, barrier layer, and adhesive layers may be colored. Such coloring includes, but is not limited to, white, black, red, yellow, blue, orange, green, violet, and mixtures thereof. Color may be imparted according to the present invention through dying, pigmentation, and printing. Colorants used according the present invention include dyes and inorganic and organic pigments. The dyes include, but are not limited to, anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like. Inorganic pigments include, but are not limited to, titanium dioxide (white), carbon black (black), iron oxides (red, yellow, and brown), chromium oxide (green), ferric ammonium ferrocyanide (blue), and the like. Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

The absorbent article may include other known materials, layers, and additives, such as, foam, net-like materials, perfumes, medicaments or pharmaceutical agents, moisturizers, odor control agents, and the like. The absorbent article can optionally be embossed with decorative designs.

The absorbent article may be packaged as unwrapped absorbent articles within a carton, box or bag. The consumer withdraws the ready-to-use article as needed. The absorbent article may also be individually packaged (each absorbent article encased within an overwrap).

### Method of using the Adhesive

The adhesive of the present invention may be applied to a garment facing side of the barrier layer for securing the napkin or liner to the microfiber containing garment during use. The adhesive may be applied as strips, swirls, or waves, and the like. The adhesive may be covered with removable release paper so that the adhesive is covered by the removable release paper prior to use. The release paper can be formed from any suitable sheet-like material that adheres with sufficient tenacity to the adhesive to remain in place prior to use but which can be readily removed when the absorbent article is to be used. Optionally, a coating may be applied to release paper (or the packaging contacting the adhesive) to improve the ease of removabilty of the release strip (or packaging) from the adhesive. US Patent no. 5,181,610 to Quick et al. provides examples of coated packages used in lieu of release liners in certain embodiments, which patent is herein incorporated by reference in its entirety.

From the foregoing description, one skilled in the art can ascertain the essential characteristics of this invention, and without departing from the spirit and scope thereof, can make various changes and modifications. Embodiments set forth by way of illustration are not intended as limitations on the variations possible in practicing the present invention.

### EXAMPLE 1

Example 1 is an example of two adhesives of the present invention (Inventive Adhesives A and B). Table 1 sets forth the ingredient listings for the two adhesives.

**Table 1**

| | **Adhesive (ingredient amount) (percent ingredient)** | |
|---|---|---|
| **Raw Material** | **Inventive Adhesive A** | **Inventive Adhesive B** |
| ESCOREZ 5400 | 2450 g (49%) | 2700 g (54%) |
| REGALREZ 1018 | 650 g (13%) | -- |
| KRYSTOL 550 | 600 g (12%) | -- |
| NYFLEX 222B | -- | 775 g (15.5%) |
| KRATON D1119 | 750 g (15%) | 1225 g (24.5%) |
| KRATON D1118 | -- | 250 g (5%) |
| SOLPRENE 1205 | 500 g (10%) | -- |
| BNX-1010 | 50 g (1%) | 50 g (1%) |
| Total | 5000 g | 5000 g |

The adhesives are prepared according to the following steps:

### Inventive Adhesive A

A suitable sized double arm bladed, sigma mixer (Teledyne-Readco, Serial 106830) and bowl is cleaned and heated to 175°C. At time zero, with blades running at 60 rpms, 400gm of ESCOREZ 5400 and 50 gm of BNX-1010 is added, followed by the slow addition of the KRATON and SOLPRENE. At the 15 minute point, all the KRATON and SOLPRENE are added and the ingredients are mixed until the mixture is uniform and homogeneous. The edges of the bowl are scraped as necessary. At the 90 minute point, the mixture is uniform and homogenous, having no visible particles. The remaining ESCOREZ 5400 is, then, slowly added with continued mixing. At the100 minute point, the KRYSTOL and REGALREZ 1018 are slowly added and the mixture is mixed until homogeneous. At the 120 minute point, the mixture is homogeneous and poured into a silicone lined container.

### Inventive Adhesive B

A suitable sized double arm bladed, sigma mixer (Teledyne-Readco, Serial 106830) and bowl is cleaned and heated to 175°C. At time zero, with blades running at 60 rpm, 400gm of ESCOREZ 5400 and 50 gm of BNX-1010 is added, followed by the slow addition of the KRATON. At the 15 minute point, all the KRATON are added and the ingredients are mixed until the mixture is uniform and homogeneous. The edges of the bowl are scraped as necessary. At the 90 minute point, the mixture is uniform and homogenous, having no visible particles. The remaining ESCOREZ 5400 is, then, slowly added with continued mixing. At the100 minute point, the NYFLEX 222B is slowly added and the mixture is mixed until homogeneous. At the 120 minute point, the mixture is homogeneous and poured into a silicone lined container.

In the above described mixing procedures, efficient mixing in the double arm sigma mixer is achieved by generating as much shear as possible. In the above procedures, a small amount of tackifying resin (ESCOREZ 5400) is blended with all of the rubber until thoroughly mixed. Once this very high viscosity blend was homogeneous, the remainder of the ingredients can be blended in and dispersed easily.

### EXAMPLE 2

Example 2 describes the T Peel test method for determining peel force of the adhesives of the present invention on cotton and microfiber fabrics.

Materials and Apparatus:
- Peel tester (Shimadzu Model AGS-X or Instrumentor's Slip/Peel Tester, Model SP1013.) capable of a 300 mm/minute peel rate, with jaws 55 mm wide to accommodate the full width of fabric and a load cell able to measure forces up to 10 N +/- 0.1 N. The Peel tester is used to measure the peel force required to remove adhesive from the fabric (i.e., cotton or microfiber).
- Recording device to calculate average peel force over range. (For Shimadzu peel tester, recording is done with "Trapezium" software ver. 1.01 for Windows). For the Slip/Peel tester, the recording is done on console provided on the Slip/Peel unit.)
- A 4.5 lb PSTC roller or PSTC automatic roll-down device (Instrumentor's PR-1000 PowerRoll (Strongsville, OH))
- Splicing tape, 70 mm wide P42, 70 mm width. (Available from H-Old S.p.a.--20010 Bareggio--MI--Italy--Via Monte Nero, 35
- Cotton fabric: Style 400: 100% Cotton Print Cloth, Scoured, Bleached, Desized, Thread Count per inch: 78 x 78, Width: 44", having a basis weight of 102 g/m²
- Microfiber fabric: FG B CLON60TN STD LX04 (95% polyamide and 5% elastane (LYCRA)) supplied by: Brugnoli Giovanni S.p.A. Via C. Ferrini, 8 - 21052 - Busto Arsizio (Varese) - Italy, having a thickness is 0.48 mm and a basis weight of 170 g/m² ±5%.

Microfiber fabric sample preparation:
- The above Brugnoli microfiber fabric has a smooth side and rough side. The rough side is characterized by a regular patterned grid, while the smooth side has relatively very little pattern. The smooth side is used as the test-side.
- A piece of microfiber fabric, about 70 mm wide by 175 mm long (3"x 7"), is cut from the Brugnoli microfiber fabric.
- The microfiber fabric stretches and must be reinforced by the splicing tape backing. A piece of splicing tape, about 70 mm wide by 190 mm long (3" x 7.5"), is cut for the backing and is laid flat on a table, sticky side up.
- The cut piece of microfiber fabric is carefully laid on top of the splicing tape with the test-side up so as not to wrinkle or stretch the microfiber fabric.
- The microfiber fabric is then firmly embedded into the splicing tape using a PSTC roller. To facilitate handling of the tape embedded microfiber fabric, the splicing tape is sized to so that a small amount of the splicing tape exceeds the length of the microfiber fabric for folding the excess tape on each lengthwise end over the lengthwise ends of the microfiber fabric.

### Cotton fabric sample preparation:

- A piece, about 70 mm wide by 175 mm long (3"x 7"), is cut from the cotton fabric. No splicing tape is use with the cotton fabric sample.

### Preparation of the Adhesive:

- An adhesive of the present invention is slot-die coated with a commercially available May Coatings CL-300 coater onto a polyester film, with the side opposite the film covered with a release liner (covering the adhesive). The adhesive coated such that the coat weight of the adhesive is 20 g/m² coat weight.
- A strip, about 25 mm wide x 300 mm long (1" x 12") is cut from the film, adhesive and release liner composite.

### Test Sample (adhesive and fabric) preparation and testing:

1. The release liner peeled back and removed from half of the film, adhesive and release liner composite strip.
2. The exposed adhesive is gently placed on the fabric for testing (i.e., the cotton fabric or the microfiber fabric)
3. The adhesive is pressed onto the fabric using the PSTC roller. The pressing process uses two passes. The strip is then rolled at 300 mm per minute using the automated roll-down device.
4. Within one minute of rolling the fabric test sample, one end of each test fabric sample is inserted into the peel tester machine and peeling is started at a rate of 300 mm per minute (12"/min).
5. The tail of the fabric test sample during the peel is positioned so as to maintain an angle of about 90 degrees.
6. The average peel force is determined by the Peel tester by measuring the peel force over a distance of at least 100 mm (4"). The average peel force (in newtons) is noted from the display of the Peel tester machine.
7. Repeat steps 1 through 6 for each of cotton and microfiber fabric sample.
8. The peel strength was tested on 5 cotton fabric samples for each tested adhesive, recorded and averaged.
9. The peel strength was tested on 5 microfiber fabric samples for each tested adhesive, recorded and averaged.

The adhesives tested included: Inventive Adhesive A; Inventive Adhesive B; Commercial Adhesive A; and Commercial Adhesive B.

The average peel strength results for each tested adhesives, using the above T-Peel procedure are shown in Table 2.

### EXAMPLE 3

Example 3 describes a test procedure for determining the rheological properties of a hot melt pressure sensitive adhesive at constant temperature, Frequency, and Shear Stress.

### Materials and Apparatus:

a. Malvern Kinexus Pro Rheometer (model # KNX5001) with 20 mm parallel plate fixture controlled by Kinexus rSpace software (version 1.60.1731.0).
b. Peltier temperature control (part # KNX-2001).

### Sample:

• About 10-15g sample of the adhesives of the present invention and commercial available adhesives.

### Procedure:

- The temperature setting on the rheometer is set to 100°C
- The 20 mm parallel plate fixture is installed and initialized (i.e., heat and equilibrate the top plate temperature).
- The plates are then separated and 10-15 grams of the sample is placed in the center of the heated bottom plate.
- The upper parallel plate is placed into contact with the sample and set a 1 mm gap.
- Excess sample is trimmed (i.e., removed) using tongue depressors.
- A sample enclosure is installed.
- The standard Kinexus procedure for shear stress controlled, constant frequency testing is initiated.
- The start and stop temperatures are entered when prompted by the rheometer.
- The heating rate (°C per minute) is entered when prompted by the rheometer
- The test shear stress is entered when prompted by the rheometer.
- The test frequency is entered when prompted by the rheometer.
- The test is started and the elastic (storage) modulus (G'), viscous (loss) modulus (G"), complex modulus (G*) and tan delta calculated and reported by the rheometer after 5 minutes.

The above test procedure is run over a range of temperatures from 125°C to -10°C at a rate of 2°C per minute, at a constant test frequency of 6.3 radians/second and at a constant test shear stress of 1000 Pascals to determine complex elastic modulus (G*'), storage modulus (G'), viscous loss modulus (G") and glass transition (Tg) values. The rheological measurements of the above tests are shown in Table 2, and indicate that, when measured at 25°C and 6.3 rad./sec., the Inventive Adhesive A has a much lower complex modulus (G*), storage modulus (G') and loss modulus (G") than Inventive Adhesive B and the commercial adhesives. And, the glass transition (Tg) temperature for Inventive Adhesive A is much lower than that of Inventive Adhesive B and the Commercial Adhesives A and B.

**Table 2**

| **Adhesive** | Average Peel Strength on Microfiber (N) | Average Peel Strength on Cotton (N) | G' at 25°C and 6.3 rad./sec. | G" at 25°C and 6.3 rad./sec. | G* at 25°C and 6.3 rad./sec. | Tg (deg C) |
|---|---|---|---|---|---|---|
| Inventive Adhesive A | 3.38 | 7.06 | 3500 Pa | 3700 Pa | 5000 Pa | -2 |
| Inventive Adhesive B | 2.70 | 3.78 | 50000 Pa | 47000 Pa | 69000 Pa | 6 |
| Commercial Adhesive A¹ | 1.86 | 3.68 | 25000 Pa | 31000 Pa | 39000 Pa | 7 |
| Commercial Adhesive B² | 1.81 | 3.48 | 27000 Pa | 27000 Pa | 38000 Pa | 5 |

| | | | | | | |
|---|---|---|---|---|---|---|
| ¹ BOSTIK H 20028 adhesive (Bostik Nederland B. V., Zeggeveld 10, 4705 RP Roosendaal, Netherlands) ² NW-1042 (H.B. Fuller, Guangzhou, China) | | | | | | |

## Claims

1. A disposable absorbent article for personal hygiene, the article having a wearer-facing surface and a garment facing surface, the garment facing surface comprising an adhesive,
wherein the adhesive has, as measured by DMA testing method described in Example 3: a) a complex modulus, at 25°C, a shear stress of 1000 Pa. and a frequency of 6.3 rad/sec., ranging from about 1000 to about 20000 Pa; b) a glass transition (Tg) temperature, at a frequency of 6.3 rad/sec. and shear stress of 1000 Pa., of less than about 10°C; or c) a combination thereof, or
wherein the adhesive comprises a first polymer wherein the first polymer is a styrenic block copolymer comprising:
i. a styrenic tri-block polymer; and
ii. a styrenic di-block polymer
to form a styrenic tri-block/diblock polymer mixture.

2. The disposable absorbent article of claim 1, wherein the adhesive has, as measured by DMA testing method described in Example 3: a) a complex modulus, at 25°C, a shear stress of 1000 Pa. and a frequency of 6.3 rad/sec., ranging from about 1000 to about 20000 Pa; b) a glass transition (Tg) temperature, at a frequency of 6.3 rad/sec. and shear stress of 1000 Pa., of less than about 10°C; or c) a combination thereof.

3. A method of adhering an article or substrate to a microfiber, comprising the steps of:
a) providing a microfiber; and
b) attaching to the microfiber an article or substrate containing an adhesive such that the article or substrate is attached to the microfiber by the adhesive, wherein the adhesive has, as measured by DMA testing method described in Example 3: i) a complex modulus, at 25°C, a shear stress of 1000 Pa. and a frequency of 6.3 rad/sec., ranging from about 1000 to about 20000 Pa; b) a glass transition (Tg) temperature, at a frequency of 6.3 rad/sec. and shear stress of 1000 Pa., of less than about 10°C; or iii) a combination thereof.

4. The disposable article of claim 2 or the method of claim 3 wherein the complex modulus, at 25°C, ranges from about 1000 to about 15000 Pa, or wherein the complex modulus, at 25°C, ranges from about 2000 to about 10000 Pa.

5. The disposable article or method of any one of claims 2-4 wherein the glass transition temperature is less than about 5°C, or
wherein the glass transition temperature is less than about 2°C.

6. The disposable article or method of any one of claims 2-5 wherein the adhesive comprises a first polymer.

7. The disposable article or method of claim 6, wherein the first polymer is a styrenic block copolymer.

8. The disposable article or method of claim 7, wherein the styrenic block copolymer comprises a styrenic tri-block polymer.

9. The disposable article or method of claim 8 wherein the styrenic block copolymer further comprises a styrenic di-block polymer, forming a styrenic tri-block/diblock polymer mixture.

10. The disposable absorbent article of claim 1, wherein the adhesive comprises a first polymer wherein the first polymer is a styrenic block copolymer comprising:
i. a styrenic tri-block polymer; and
ii. a styrenic di-block polymer
to form a styrenic tri-block/diblock polymer mixture.

11. The disposable article or method of claim 9 or claim 10 wherein the styrenic di-block polymer is present at a concentration of at least 50%, by weight of the styrenic tri-block/diblock polymer mixture, or
wherein the styrenic di-block polymer is present at a concentration of at least 60%, by weight of the styrenic tri-block/diblock polymer mixture, or
wherein the styrenic di-block polymer is present at a concentration of at least 70%, by weight of the styrenic tri-block/diblock polymer mixture.

12. The disposable article or method of any one of claims 6-11 wherein the first polymer has a melt index of greater than about 12 grams per 10 minutes as measured by ASTM Method D 1238, Condition G.

13. The disposable article or method of any one of claims 6-12 wherein the adhesive further comprises a second polymer.

14. The disposable article or method of claim 13 wherein the second polymer has a melt index less than about about 12 grams per 10 minutes as measured by ASTM Method D 1238, Condition G and/or
wherein the second polymer is a styrene-butadiene polymer.

15. The disposable article or method of any preceding claim, wherein the article is an incontinence product, panty liner or feminine napkin.
